# EUROPEAN PATENT SPECIFICATION

(11) **EP 0 367 554 B1**
(45) Date of publication and mention of the grant of the patent: **06.09.1995**
(21) Application number: 89311220.1
(22) Date of filing: 31.10.1989
(51) Int. Cl.: C07K 14/00, C07K 1/12, A61K 38/16

(54) **Insulin binders and inhibitors of insulin binding**
Insulinbindende Wirkstoffe und Hemmer der Insulinbindung
Agents liant l'insuline et inhibant la liaison de l'insuline

(30) Priority: 31.10.1988 JP 273139/88; 21.09.1989 JP 243513/89
(43) Date of publication of application: 09.05.1990
(73) Proprietor: TAKARA SHUZO CO. LTD., Fushimi-ku Kyoto 612 (JP)
(72) Inventor: Yoshihito, Yaoi, Yokohama-shi Kanagawa-ken (JP); Kahoko, Hashimoto, Meguro-ku Tokyo-to (JP); Kazuhiko, Takahara, Otsu-shi Shiga-ken (JP); Fumitsugu, Hino, Kusatsu-shi Shiga-ken (JP); Ikunoshin, Kato, Uji-shi Kyoto-fu (JP)
(74) Representative: Geering, Keith Edwin

(56) References cited:
- CELL STRUCTURE AND FUNCTION, vol. 13, no. 6, December 1988, page 626, abstract no. 1D-1548, Japan Society for Cell Biology (JSCB), Kyoto, JP; K. TAKAHARA et al.: "Insulin-binding properties of extracellular matrix proteins"
- CHEMICAL ABSTRACTS, vol. 115, no. 1, 8th July 1991, page 135, abstract no.1323n, Columbus, Ohio, US; Y. YAOI et al.: "Insulin binds to type V collagen with retention of mitogenic activity", & EXP. CELL. RES. 1991,194(2), 180-5
- CHEMICAL ABSTRACTS, vol. 113, no. 15, 8th October 1990, page 264, abstract no.128330b, Columbus, Ohio, US; Y. YAOI et al.: "Primary structure of the heparin-binding site of type V collagen", & BIOCHIM. BIOPHYS. ACTA 1990,1035(2), 139-45

## Description

This invention relates to a protein or polypeptide which has insulin-binding activity, to a composition for use as an insulin-binding carrier which has, as its active component, said protein or polypeptide, and also to an inhibitor of the binding of insulin with collagen.

To date, proteins and polypeptides known to bind with insulin have included insulin receptors located on the surfaces of cells.

Also, as non-receptor proteins, we have earlier discovered that fibronectin, laminin, chondronectin, fibrinogen, vitronectin, and fibrin can bind with insulin, and have suggested a composition for use as an insulin-binding carrier (Japanese Laid-Open Patent Application No. 85934/89).

It has been known for a long time that both insulin-like growth factor (IGF-I, or somatomedin C), which has a structure that closely resembles that of insulin, and IGF-II are bound by certain binding proteins that are not receptors, and that these IGF-binding proteins can be extracted from blood and the like. They have recently been purified to homogeneity. Unlike the situation with insulin, IGF forms complexes of high molecular weight with binding proteins in the blood, and generally large amounts of such complexes are found in inactive form in living organisms. Also, when it form a complex, homeostatis is affected, as IGF in the body is stabilized and its biological half-life increases.

The present invention provides a novel insulin-binding polypeptide and a composition for use as an insulin-binding carrier, both of which have insulin-binding activity and also an inhibitor of the binding of insulin to the said insulin-binding carrier.

Insulin-binding polypeptide of this invention has in its structure at least the amino acid sequence represented by the following Formula (I): 
-Gly-Gly-Arg-Gly-Thr-Hyp-Gly-Lys-Hyp-
Gly-Pro-Arg-Gly-Gln-Arg-Gly-Pro-Thr-
Gly-Pro-Arg-Gly-Glu-Arg-Gly-Pro-Arg-
Gly-Ile-Thr-Gly-Lys-Hyp-Gly-Pro-
............... [ I ]
In considering the problem of insulin binding we felt that there might be proteins which have insulin-binding activity and that there might be proteins which can be used as carriers of insulin. We felt that such a protein or polypeptide might participate in the regulation of the homeostasis of insulin, which acts both as a growth factor and as a hormone; when insulin is administered to patients with a variety of disorders, of which diabetics are representative, it might be possible to use such a protein or polypeptide as part of a drug delivery system or in a new pharmaceutical preparation designed to act on the target of the insulin, and with longer-term drug effectiveness, greater safety, and no immunogenicity, by which means the efficacy of the insulin would be enhanced and side effects would be few. For example, it might be possible to suggest reagents for biochemical research that could accelerate growth, cosmetics that could enhance growth of skin cells (preventing ageing of the skin), drugs given percutaneously and by other routes that accelerate the healing of wounds (including surgical incisions),and drugs for diabetes with longer-term effectiveness, with few side effects.

We also felt that it might be possible to inhibit the binding of insulin with such an insulin-binding carrier, in which case it would be easy to regulate the expression of the effects of insulin in the body and to make use of a drug-delivery system such as mentioned above.

We have found by research that collagen type I, type II, type III, type IV, and type V can bind with insulin, and that collagen V has a specific insulin-binding site. We also found that the binding of insulin with the said collagen can be inhibited by insulin A chain, insulin B chain, and a polypeptide of decomposed insulin B chain which has a collagen-binding active site.

Accordingly the present invention also provides the use of a composition for the preparation of an insulin-binding carrier which has as its active component at least one kind of protein or polypeptide selected from the group of collagen type I, type II, type III, type IV, type V and polypeptides of decomposed collagen V which contain the insulin-binding active site; and further provides the use as active component of at least one kind of polypeptide selected from the group consisting of insulin A chain, insulin B chain and polypeptides of decomposed insulin B chain which contain the collagen-binding active site in an inhibitor of the binding of insulin with collagen.

Collagen is the main protein which makes up connective tissue, and the collagen molecule is composed of three alpha chains intertwined in a right-handed helix. The structure of the alpha chains varies somewhat in different tissues, and collagen is classified as type I, type II, type III, type IV, or type V depending on these differences. The peptide hormone made of insulin A chain and B chain is known to have a variety of physiological functions.

We used peroxidase-labelled insulin to identify proteins with insulin-binding activity; a protein sample is allowed to bind with said insulin, and it is then possible to detect insulin-binding activity by measurement of the peroxidase activity of the resultant complex. When a number of different components of the living body were investigated, it was found that collagen type I, type II, type III, type IV, and type V, (particularly type V) all had strong insulin-binding activity.

By the use of physical, chemical, and enzymatic methods, the collagen molecule was fragmented, and the various fragments were separated and analyzed with the use of the peroxidase-labelled insulin mentioned above, by which means it was possible to identify the polypeptide that had insulin-binding activity, that is, the insulin-binding site of collagen. We discovered that the insulin-binding active side of collagen type V, when collagen type V was decomposed, was in the α₁ chain and in a polypeptide with the molecular mass of 30 kDa that was produced when, for example, the collagen type V was decomposed with cyanogen bromide. This polypeptide also had heparin-binding activity, and could be effectively purified on a heparin column. Next, we decomposed the 30-kDa polypeptide to identify the site of insulin binding in it. The 30-kDa polypeptide was separated into a 16-kDa polypeptide and a 6-kDa polypeptide by treatment with endoprotease-Glu-C (Protease V8, Boehringer Mannheim-Yamanouchi) and by applying the digest obtained to a heparin column and then an ODS reverse-phase column, or the like. These two smaller polypeptides had both heparin-binding activity and insulinbinding activity, but their binding activities were less than these activities of the 30-kDa polypeptide. The 16-kDa polypeptide was further decomposed by the use of lysyl endopeptidase (Wako Pure Chemical Industries, Ltd.) into three smaller polypeptides, and these polypeptides were separated by the use of an ODS reverse-phase column. The amino acid sequence of collagen type V has not yet been determined completely, but the amino acid sequences of the various polypeptides obtained from it and the amino acid sequence of the 6-kDa polypeptide have been determined. By comparative analysis of these with the amino acid sequence of collagen type XI (J. Biol. Chem., 263, 17159-17166, 1988), which resembles collagen type V, it was possible to determine the amino acid sequence of the 30-kDa polypeptide. By this procedure, it was found that the 6-kDa polypeptide is bound to the C-terminal of the 16-kDa polypeptide, and that the amino acid sequence in the portion where these two polypeptides are bound together is the amino acid sequence shown by the Formula (I). By cleavage of the 16-kDa polypeptide and the 6-kDa polypeptide, both the insulin-binding activity and the heparin-binding activity decreased, so the C-terminal portion of the 16-kDa polypeptide and the N-terminal portion of the 6-kDa polypeptide, that is, the amino acid sequence of the Formula (I), was an important sequence for the insulin-binding activity and heparin-binding activity of collagen type V.

As the polypeptide from decomposed collagen type V of this invention, any polypeptide is suitable provided that it contains the amino acid sequence of the Formula (I), and the polypeptide can be prepared by chemical synthetic methods or genetic engineering methods as well as by use of chemical methods such as cyanogen bromide decomposition, or enzymatic methods such as the use of endopeptidase Glu-C.

For the investigation into an inhibitor of the binding of insulin with collagen, peroxidase-labelled insulin can again be used. The sample and the said insulin can be allowed to react with collagen type V at the same time, and the peroxidase activity of the complexes with collagen can be measured after this reaction, by which means the inhibition by the sample of the binding of insulin with collagen can be evaluated. We have found that insulin A chain, B chain, and polypeptides of decomposed insulin B chain can inhibit the binding of insulin with collagen.

To identify the collagen-binding active site of insulin, insulin was fragmented enzymatically, and these fragments were allowed to react with collagen type V at the same time as peroxidase-labelled insulin, by which means it was possible to identify the fragment of insulin that bound with collagen. Insulin chain B was decomposed with the use of endopeptidase Glu-C, and from among the three kinds of fragments that were produced, fragments containing the collagen-binding active site were identified.

As the polypeptides from decomposed insulin B chain of this invention, any polypeptides are suitable provided that they contain the active sites of collagen-binding activity of these polypeptides, and the polypeptide can be prepared by chemical synthetic methods or genetic engineering methods as well as by the chemical or enzymatic decomposition of insulin B chain.

By the method of inhibition of the binding of insulin with collagen that makes use of insulin A chain, insulin B chain, or the polypeptides of decomposed B chain, it is possible to regulate in detail the use of a drug delivery system that involves a composition for use as an insulin-binding carrier that makes use of collagen. Also, it would be possible to use such an inhibitor for the prevention and treatment of insulin dysfunction such as the transitory diabetes that may occur as a response to the invasiveness of surgery.

The insulin-binding carrier and inhibitor of the binding of insulin with collagen of this invention can themselves be in the form of, or can be given in ordinary pharmaceutical preparations in the form of, for example, a solution, lotion, ointment, gel, tablet, or capsule, or can be a form that is attached, bound, or contained in a solid substrate, percutaneous apparatus, implant piece and the like.

The invention will be explained in more detail with reference to the following examples. However, this invention is not to be taken to be restricted to these examples.

### Example 1. Insulin-binding carrier.

First, samples of bovine collagen type I, type II, type III, type IV, and type V (Koken) were separately dissolved to a concentration of 180 µg/ml in a solution of 150 mM acetic acid which contained 50 mM NaCl. Each preparation was diluted stepwise, and a 50-µl portion of each was put into a well of a 96-weel micro-plate. This plate was left for 2 hours at 37°C. Then each well was washed with phosphate-buffered saline that did not contain bivalent metal ions [PBS⁽⁻⁾], and then blocking was done with the use of bovine serum albumin (BSA). Next, peroxidase-labelled insulin (Sigma) was dissolved in PBS⁽⁻⁾ to a concentration of 5 µg/ml, and 50 µl of this solution was put into each well. The plate was left for 2 hours at 37°C to allow the reaction to proceed, and then peroxidase-labelled insulin that had not formed a complex was washed away. The peroxidase activity of insulin binding and collagen binding was measured with 2,2′-azino-bis-(3-ethylbenzothiazoline-6-sulfonic acid) (ABTS) and hydrogen peroxide as substrates.

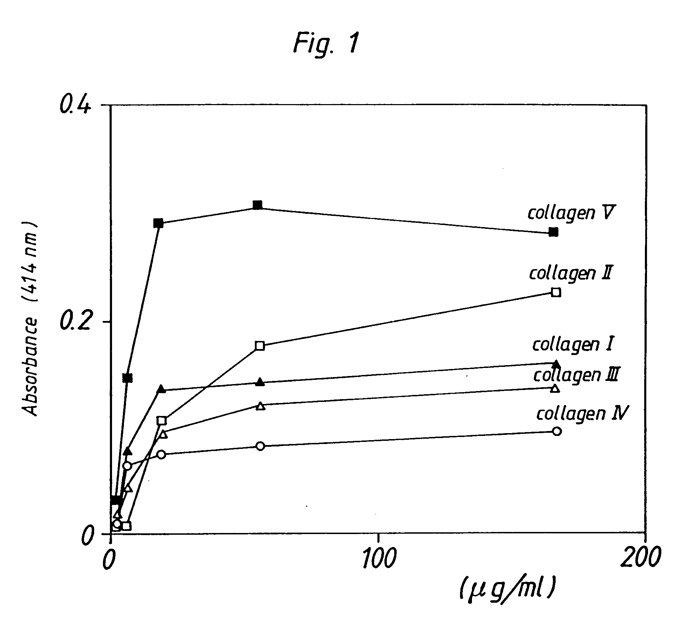

The results showed that each type of collagen could bind with insulin. These results are shown in Fig. 1. There, the collagen concentration of each type of collagen (in micrograms per milliliter, shown as the X axis) was 180, 60, 20, 6.6, or 2.2 µg/ml, and the absorbance at 414 nm (shown as the Y axis) was used as an index of peroxidase activity, which reflected insulin binding.

### Example 2. Insulin-binding polypeptide

The collagen type V mentioned above was dissolved to a concentration of 1 mg/ml, and 10 µl of this solution was treated by SDS-polyacrylamide gel electrophoresis. After transfer to a nitrocellulose membrane by Western blotting, the membrane was immersed in PBS that contained 5 µg/ml peroxidase-labelled insulin and 0.05% Tween 20 at room temperature for 2 hours to allow the reaction to proceed. The peroxidase activity that was bound to the nitrocellulose membrane was detected with 4-chloro-1-naphthol and hydrogen peroxide as substrate.

The results showed that peroxidase-labelled insulin bound with the α₁ chain of collagen type V.

Next, collagen type V was dissolved in 70% formic acid that contained 20 mg/ml cyanogen bromide to a concentration of 5 mg/ml, and the reaction was allowed to proceed for 24 hours at room temperature, resulting in fragmentation of the collagen type V. The fragments of collagen type V were treated by electrophoresis as described above, and then treated with peroxidase-labelled insulin. The fragment with a molecular mass of about 30 kDa (30-kDa polypeptide) was found to have the insulin-binding activity.

The collagen type V treated above with cyanogen bromide was separated by use of high-pressure liquid chromatography (HPLC) with a heparin column (TSK Gel Heparin 5PW, Tosoh Corp.) equilibrated with PBS⁽⁻⁾ that contained 2 M urea. An excess of solvent was used to wash the column, and then the 30-kDa polypeptide mentioned above was eluted with solvent to which 1 M NaCl had been added. Next, water was used to remove the solvent from the eluant by dialysis, and the resultant dialysate was lyophilized. This product was dissolved in 0.2 M ammonium biacetate (pH 4.0) to a concentration of 0.1%, and to 1 ml of this solution was added 20 µg of endoprotease Glu-C. The mixture was allowed to react overnight at 37°C. Then the reaction mixture was applied to a heparin column equilibrated with threefold-diluted PBS⁽⁻⁾, and the column was washed with an excess of solvent before the adsorbed substance was eluted with solvent that contained 0.5 M NaCl. The eluant was treated by HPLC on an ODS reverse-phase column equilibrated with 0.1% trifluoroacetate. By the use of a 0-60% gradient of acetonitrile, two components were fractionated. The molecular masses of the two components were 16 kDa and 6 kDa.

Insulin-binding activity was tested by the dot-blotting method with use of peroxide-labelled insulin, and both fragments had insulin-binding activity that was somewhat weaker than that of the 30-kDa fragment.

In order to identify the amino acid sequence of the 30-kDa polypeptide, the 16-kDa polypeptide was decomposed by the use of lysyl endopeptidase. Here, the lysyl endopeptidase was dissolved in 50 mM ammonium hydrogen-carbonate solution (pH 7.8) to a concentration of 10 µg/ml, and the 16-kDa polypeptide was digested overnight at 37°C. The fragments were treated as described above by HPLC on an ODS reverse-phase column, and three fragments (L-1, L-2, and L-3) were obtained. Next, these fragments were lyophilized and the solvent was removed.

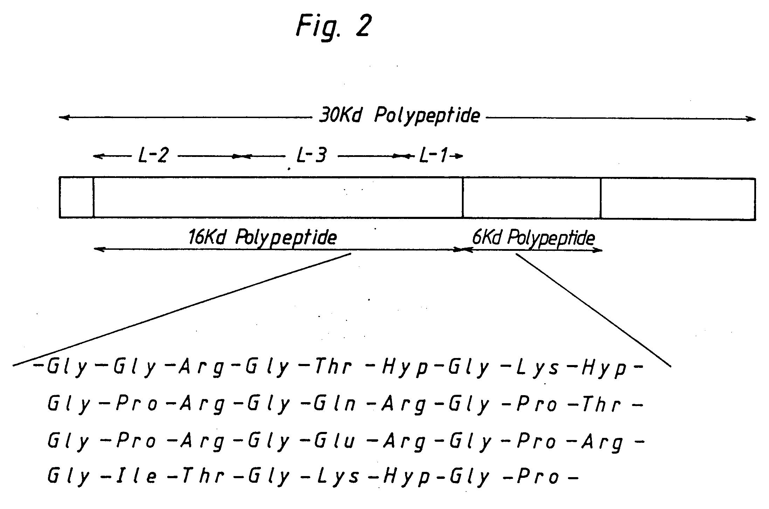

The amino acid sequences of the three fragments, L-1, L-2, and L-3, and the 6-kDa polypeptide were analyzed with use of a protein sequencer 477A (Applied Biosystems, Inc.). By comparison of the amino acid sequences found here with the amino acid sequence of collagen XI mentioned above, the structure of the 30-kDa polypeptide and its partial amino acid sequence were both determined, by comparison of the sites of the 30-kDa polypeptide cleaved by endoprotease Glu-C and pysyl endopeptidase, to be as shown in Figure 2. Thus, Figure 2 shows the structure and the partial amino acid sequence of the 30-kDa polypeptide.

### Example 3. Inhibitor of the binding of insulin with collagen

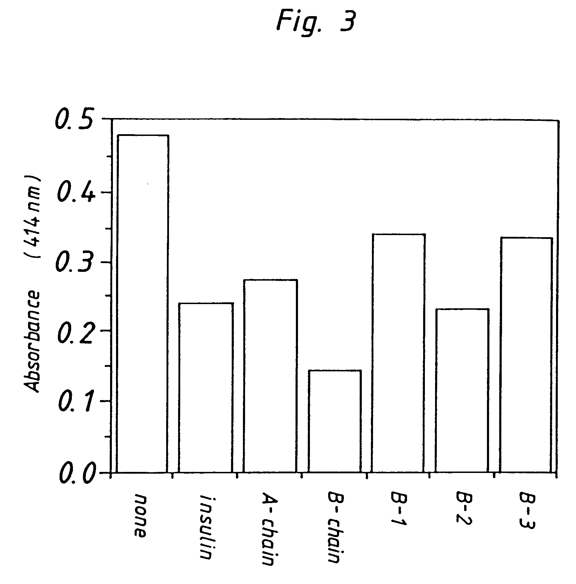

By the method described above, wells of a 96-well microplate were coated with collagen type V, and blocking was done with BSA. Then, to each well, 5 µg/ml peroxidase-labelled insulin was added, and then 50 µl of PBS⁻ that contained insulin, insulin A chain, or insulin B chain (Sigma) at a concentration of 100 µg/ml was added. The plate was then left for 2 hours at 37°C. After this time, unbound substances were removed by washing of the wells, and the activity of bound peroxidase was assayed with the use of ABTS and hydrogen peroxidase as substrates. Results showed that insulin A chain and insulin B chain inhibited the binding of insulin with collagen. The results for the polypeptides of decomposed insulin B chain are also shown in Figure 3, which shows insulin and compounds related to insulin (on the X axis) and their ability to inhibit the binding of collagen in terms of peroxidase activity, evaluated as the absorbance at 414 nm (on the Y axis).

Next, to a 0.1% solution of insulin B chain in 1 ml of 50 mM phosphate buffer (pH 7.5), 20 µg of endoprotease Glu-C was added, and the mixture was allowed to react for 2 hours at 37°C. The reaction mixture was applied to an ODS reverse-phase column, and fractionated by HPLC. The fragments of insulin B chain were eluted from the reverse-phase column in the order of ¹Phe-Val-Asn-Glu-His-Leu-Cys-Gly-Ser-His-Leu-Val-¹³Glu (B-1), ¹⁴Ala-Leu-Tyr-Leu-Val-Cys-Gly-Glu²¹ (B-2), and ²²Arg-Gly-Phe-Phe-Tyr-Thr-Pro-Lys-³⁰Thr (B-3). The ability of each of these three polypeptides to inhibit the binding of insulin with collagen was assayed by the methods described above, and it was found that each of these polypeptides could inhibit the binding of insulin with collagen. The results are shown in Figure 3.

As is seen by the above description, the novel composition for use as an insulin-binding carrier and the insulin-binding polypeptide of this invention have the ability to bind insulin, and the results of studies of these substances suggested that they could be used in newly discovered applications when insulin is used in the fields of biochemistry and medicine by making possible the more effective use of insulin. In particular, they would be useful in new preparations of insulin and as part of drug delivery systems. Also, they might be used to control the growth of cells (including cancer cells) that require insulin as a growth factor.

Also, the inhibitors of the binding of insulin with collagen of this invention are novel uses of insulin components and help ensure that the insulin-binding polypeptide will be effective and useful in applications of biochemical and medical significance in thc field of biochemistry and medicine in which insulin is used. In particular, they would be useful in new preparations of insulin and when drug delivery systems are being employed. Also, they could be useful in the prevention and treatment of transitory insulin deficiency in the diabetes that arises as a response to the invasiveness of surgery.

In the accompanying drawings, Figure 1 shows the insulin binding of the various kinds of collagen; Figure 2 is a graph that shows the structure and the partial amino acid sequence of the 30-kDa polypeptide; and Figure 3 is a graph of the ability of insulin and related compounds to inhibit insulin binding and collagen binding.

## Claims

1. A polypeptide which has insulin-binding activity and which has as at least part of its structure the amino acid sequence of the following Formula [I].

-Gly-Gly-Arg-Gly-Thr-Hyp-Gly-Lys-Hyp-
Gly-Pro-Arg-Gly-Gln-Arg-Gly-Pro-Thr-
Gly-Pro-Arg-Gly-Glu-Arg-Gly-Pro-Arg-
Gly-Ile-Thr-Gly-Lys-Hyp-Gly-Pro-
................ [I]

2. A polypeptide according to claim 1 which has molecular mass of 30 kDa and which is obtainable by the decomposition of collagen type V by treatment with cyanogen bromide.

3. The use in the preparation of an insulin-binding carrier of at least one protein or polypeptide selected from collagen types I, II, III, IV and V and polypeptides of decomposed collagen V which contain the insulin binding active site.

4. The use as active component of at least one polypeptide selected from insulin A chain, insulin B chain, and polypeptides of decomposed insulin B chain which contain the collagen-binding site in the preparation of an inhibitor of the binding of insulin with collagen.

## Patentansprüche

1. Polypeptid, welches eine Insulinbindungsaktivität aufweist und welches zumindest als Teil seiner Struktur die Aminosäuresequenz der folgenden Formel (I): 
-Gly-Gly-Arg-Gly-Thr-Hyp-Gly-Lys-Hyp-
Gly-Pro-Arg-Gly-Gln-Arg-Gly-Pro-Thr-
Gly-Pro-Arg-Gly-Glu-Arg-Gly-Pro-Arg-
Gly-Ile-Thr-Gly-Lys-Hyp-Gly-Pro-
................ [I]
 besitzt.

2. Polypeptid nach Anspruch 1, welches eine Molekularmasse von 30 kDa besitzt und welches durch Zersetzung von Kollagen des Typus V durch Behandlung mit Cyanbromid erhältlich ist.

3. Verwendung von mindestens einem Protein oder Polypeptid, gewählt aus Kollagen der Typen I, II, III, IV und V, und Polypeptiden von zersetztem Kollagen V, welche die aktive Insulinbindungsstelle enthalten, bei der Herstellung eines Insulin-bindenden Trägers.

4. Verwendung von mindestens einem Polypeptid, gewählt aus Insulin A-Kette, Insulin B-Kette; und Polypeptiden von zersetzter Insulin B-Kette, welche die Kollagenbindungsstelle enthalten, als aktive Komponente bei der Herstellung eines Inhibitors für die Bindung von Insulin mit Kollagen.

## Revendications

1. Polypeptide qui possède une activité de liaison à l'insuline et dont la structure comporte en partie au moins la séquence d'acides aminés répondant à la formule [I] suivante:

-Gly-Gly-Arg-Gly-Thr-Hyp-Gly-Lys-Hyp-
Gly-Pro-Arg-Gly-Gln-Arg-Gly-Pro-Thr-
Gly-Pro-Arg-Gly-Glu-Arg-Gly-Pro-Arg-
Gly-Ile-Thr-Gly-Lys-Hyp-Gly-Pro-

.................. [I]

2. Polypeptide selon la revendication 1 qui possède une masse moléculaire de 30 KDa et qui peut être obtenu par décomposition du collagène de type V par traitement au bromure de cyanogène.

3. Utilisation d'au moins une protéine ou d'un polypeptide choisi parmi le collagène des types I, II, III, IV et V et des polypeptides issus de la décomposition du collagène de type V contenant le site actif de liaison à l'insuline dans la préparation d'un véhicule liant l'insuline.

4. Utilisation à titre de principe actif d'au moins un polypeptide choisi parmi la chaîne A de l'insuline, la chaîne B de l'insuline, et de polypeptides issus de la décomposition de la chaîne B de l'insuline contenant le site liant le collagène dans la préparation d'un inhibiteur de la liaison de l'insuline au collagène.
